# EUROPEAN PATENT APPLICATION

(11) **EP 0 665 239 A1**
(43) Date of publication of application: **02.08.1995**
(21) Application number: 94200176.9
(22) Date of filing: 26.01.1994
(51) Int. Cl.: C07K 14/47

(54) **Peptide derivatives of bactenecin endowed with antibacterial activity**

(71) Applicant: CONSIGLIO NAZIONALE DELLE RICERCHE, I-00185 Roma (IT); CONSORZIO PER LO SVILUPPO INTERNAZIONALE DELL'UNIVERSITA DI TRIESTE, I-34127 Trieste (IT)
(72) Inventor: Gennaro, Renato, c/o Univ. degli Studi di Trieste, I-34127 Trieste (IT); Skerlavaj, Barbara,, Via L. Giorgieri 1, I-34127 Trieste, (IT); Scocchi, Marco,, Via L. Giorgieri 1, I-34127 Trieste, (IT); Romeo, Domenico,, Via L. Giorgieri 1, I-34127 Trieste, (IT)
(74) Representative: Marchi, Massimo

(57) **Abstract**

A peptide of formula

Arg-Z₁-Pro-Z₂-Arg-Pro-Z₃ (I)

wherein
- Z₁: is Arg-Ile-Arg or Phe-Arg-Pro,
- Z₂: is Arg-Pro-Pro-Arg-Leu-Pro or Ile-Arg-Arg-Pro-Pro-Ile,
- Z₃: is Arg-Pro-Arg-Pro-Leu, Pro-Phe-Tyr-Pro-Pro-Phe-Arg-Pro, Arg-Pro-Arg-Pro-Leu-Pro-Phe-Pro-Arg-Pro, Pro-Phe-Tyr-Pro-Pro-Phe-Arg-Pro-Pro-Ile-Arg-Pro or Arg-Pro-Arg-Pro-Leu-Pro-Phe-Pro-Arg-Pro-Gly-Pro-Arg-Pro-Ile-Pro-Arg-Pro-Leu-Pro-Phe-Pro.
Peptides are fragments and/or derivatives of naturally occurring bactenecins. They are endowed with antibacterial activity.

## Description

This invention concerns peptides endowed with antibacterial activity.

In the last ten to fifteen years, several peptides and proteins that exert a potent antibacterial action and which participate in the defence of the host organism, have been isolated in pure form from multicellular organisms such as arthropods, amphibians and mammals.

These compounds, for which the term "antibiotics from within" has been proposed, resemble commercial antibiotics little if at all. Several uses have been proposed for these compounds, but one of the most promising is their use as food preservatives.

Research carried out in the last few years by the inventors themselves have led to the identification and characterization of two novel polypeptides, isolated from bovine neutrophils, which are rich in proline (>45%) and arginine (>20 %), and which exert a potent antibacterial action in vitro (Gennaro et al., Infect. Immun. 57: 3142-3146, 1989; Zanetti et. al., J. Cell Biol. 111: 1363-1371, 1990; Frank et. al., J. Biol. Chem. 265: 18871-18874, 1990; Skerlavaj et. al. Infect. Immun. 58: 3724-3730, 1990; Zanetti et. al., J. Immunol. 146: 4295-4300, 1991).

These polypeptides have been named bactenecins and termed Bac5 and Bac7, based on their respective molecular weights of about 5000 and 7000, respectively.

Thanks to a further study of these compounds, it has now been surprisingly found that the compounds of formula

Arg-Z₁-Pro-Z₂-Arg-Pro-Z₃ (I)

where
- Z₁: is Arg-Ile-Arg or Phe-Arg-Pro,
- Z₂: is Arg-Pro-Pro-Arg-Leu-Pro or Ile-Arg-Arg-Pro-Pro-Ile,
and
- Z₃: is Arg-Pro-Arg-Pro-Leu, Pro-Phe-Tyr-Pro-Pro-Phe-Arg-Pro, Arg-Pro-Arg-Pro-Leu-Pro-Phe-Pro-Arg-Pro, Pro-Phe-Tyr-Pro-Pro-Phe-Arg-Pro-Pro-Ile-Arg-Pro or Arg-Pro-Arg-Pro-Leu-Pro-Phe-Pro-Arg-Pro-Gly-Pro-Arg-Pro-Ile-Pro-Arg-Pro-Leu-Pro-Phe-Pro,
have an antibacterial activity which is at the least comparable to that of Bac5 or Bac7, in molar terms.

Therefore, it is a first object of this invention to provide the compounds of formula (I).

Preferred examples of compounds of formula (I) according to this invention are those wherein:
- Z₁ is Arg-Ile-Arg, Z₂ is Arg-Pro-Pro-Arg-Leu-Pro, and Z₃ is Arg-Pro-Arg-Pro-Leu-Pro-Phe-Pro-Arg-Pro;
- Z₁ is Phe-Arg-Pro, Z₂ is Ile-Arg-Arg-Pro-Pro-Ile, and Z₃ is Pro-Phe-Tyr-Pro-Pro-Phe-Arg-Pro-Pro-Ile-Arg-Pro;
- Z₁ is Arg-Ile-Arg, Z₂ is Arg-Pro-Pro-Arg-Leu-Pro, and Z₃ is Arg-Pro-Arg-Pro-Leu; and
- Z₁ is Arg-Ile-Arg, Z₂ is Arg-Pro-Pro-Arg-Leu-Pro, and Z₃ is Arg-Pro-Arg-Pro-Leu-Pro-Phe-Pro-Arg-Pro-Gly-Pro-Arg-Pro-Ile-Pro-Arg-Pro-Leu-Pro-Phe-Pro.

It is a second object of this invention to provide a process for preparing the compounds of formula (I).

Said process is preferably carried out according to the conventional technique for solid phase peptide synthesis. In particular, the chemical method known as the Fmoc method, carried out on a continuos flow, automated synthesizer (9050, Millipore), is preferred.

An example of a suitable insoluble support is the polyamide resin PepSyn™ KA (Millipore), polymerized on an inorganic matrix, preferably Kieselguhr, and to which the C-terminal amino acid is linked via a suitable linker, while the alfa-amino group is protected by the 9-Flourenyl methoxycarbonyl group (Fmoc).

The successive amino acids, also protected in a similar manner, are subjected to the condensation reaction by means of repeated cycles of deprotection in a basic environment, and coupling in the presence of suitable catalysts.

In the case of non-preactivated amino acids, examples of suitable catalysts are
- benzotriazol-1-yl-N-oxy-tris(dimethylamino)phosphonium hexafluorophosphate (BOP) coupled with 1-hydroxybenzotriazole (HOBt), and
- 2-(1H-benzotriazol-1-yl)-1,1,3,3-tetramethyluronium tetraflouroborate (HBTU).

In the case of amino acids preactivated with pentafluorophenyl esters, an example of a suitable catalyst is instead HOBt.

Arginine and tyrosine residues are preferably protected, besides on the alpha-amino group, also on the side chain, by means of acid labile groups such as, for example, 2,2,5,7,8-pentamethylchroman-6-sulphonyl (Pmc) for Arg and tert-butyl (tBu) for Tyr.

On completion of the synthesis, the desired peptide is cleaved from the solid support preferably by exposure to trifluoroacetic acid, a process which also results in the deprotection of the side chains of Arg and Tyr.

The purification of the peptide so obtained, from contaminants produced during the synthesis, can be achieved by means of HPLC with a reverse phase column (Delta Pak™ C18, Millipore) eluted with an acetonitrile/water gradient in the presence of 0.1% trifluoroacetic acid (TFA).

The amino acid composition of the peptide so produced can be easily checked via acid hydrolysis of the sample, derivatization of the free amino acids with phenylisothiocyanate so as to obtain the corresponding PTH-derivatives, and identification and quantitative determination of the latter via reverse phase chromatography.

The following examples illustrate this invention without, however, limiting it.

### Example 1

### Preparation of Peptides

For the synthesis of the peptide of formula Arg-Arg-Ile-Arg-Pro-Arg-Pro-Pro-Arg-Leu-Pro-Arg-Pro-Arg-Pro-Arg-Pro-Leu-Pro-Phe-Pro-Arg-Pro-Gly-Pro-Arg-Pro-Ile-Pro-Arg-Pro-Leu-Pro-Phe-Pro (Peptide A)
1 g of PepSyn™ KA (Millipore) resin was utilised, to which 0.09 mmole of proline, protected with the Fmoc group, had been attached by means of a 4-hydroxymethylphenoxyacetic acid linker.

Once introduced into the synthesis column, the resin was initially solvated with dimethylformamide (DMF) and then treated with 20% piperidine in DMF so as to remove the Fmoc group and to enable the attachment of the following amino acid, a phenylalanine.

The latter residue, as all subsequent residues, was introduced dissolved in DMF, in a five-fold excess (0.45 mmoles) with respect to the resin substitution, and was protected with Fmoc on its alpha-amino group.

This phenylalanine, as the other present in peptide A, as well as the single glycine, the two isoleucines, the fifteen prolines, not considering the C-terminal one attached to the resin, and the three leucines were introduced in the form activated as the pentafluorophenyl esters (OPfp), together with an equimolar quantity of 1-hydroxybenzotriazole (HOBT).

The acylation reaction between the free amine group on the growing, resin-linked peptide and the successive amino acid was protracted for 45-60 minutes, by recirculating in the column the OPfp-amino acid dissolved in DMF.

In the case of the eleven arginines, these were protected with Fmoc on the amine group, and with 2,2,5,7,8-pentamethylchroman-6-sulphonyl (Pmc) on the guanidine group.

In contrast to the other amino acids cited above, the arginines were not preactivated on the carboxylic acid group and were thus added together with equimolar quantities of the catalyst benzotriazol-1-yl-N-oxytris(dimethylamino)phosphonium hexafluorophosphate (BOP) and the catalyst HOBT, in the presence of two equivalents of N-methylmorpholine. Each acylation cycle with arginine was protracted for 45 minutes.

Each acylation was followed by Fmoc deprotection of the introduced amino acid, by means of a 20% piperidine in DMF solution.

As the Peptide A was particularly long, and in order to increase the final yield, a double coupling was effected for the last fifteen amino acids (corresponding to residues 1 to 15). In other words, the acylation stage was not followed by Fmoc deprotection, but, after washing, the acylation was repeated under the same conditions and with the same quantities of amino acid used in the preceding cycle. Only at this stage was the Fmoc deprotection carried out, and the subsequent amino acid added, also in two cycles not separated by a deprotection, until the N-terminal residue was reached.

At the end of the synthesis, the resin was washed with 20% piperidine in DMF so as to remove the Fmoc group on the last residue, and then thoroughly washed, after removal from the column, with tert-amyl alcohol, acetic acid, and again tert-amyl alcohol, before suspending it in ethyl ether. The latter was then evaporated until the resin was completely dry.

After this process the resin was resuspended for 3 hours in 95% TFA and 5% phenol, so as to cleave the peptide from the resin and deprotect the arginines from the Pmc group.

At this stage the peptide was separated from the resin by filtration, precipitated in ethyl ether, vacuum dried and resuspended in 0.1% TFA for the purification via HPLC. This was carried out on a reverse phase Delta Pak™ C₁₈ (Millipore) column, eluted at a flow rate of 2 ml/min with a 30 min, linear, 0-50% gradient of acetonitrile/water in 0.1% TFA.

The synthesis of the following peptides:
- Arg-Arg-Ile-Arg-Pro-Arg-Pro-Pro-Arg-Leu-Pro-Arg-Pro-Arg-Pro-Arg-Pro-Leu-Pro-Phe-Pro-Arg-Pro (Peptide **B**)
- Arg-Phe-Arg-Pro-Pro-Ile-Arg-Arg-Pro-Pro-Ile-Arg-Pro-Pro-Phe-Tyr-Pro-Pro-Phe-Arg-Pro-Pro-Ile-Arg-Pro (Peptide **C**), and
- Arg-Arg-Ile-Arg-Pro-Arg-Pro-Pro-Arg-Leu-Pro-Arg-Pro-Arg-Pro-Arg-Pro-Leu (Peptide **D**)
was carried out starting with 1 g of PepSyn™ KA resin with attached 0.09 mmole of proline, in the case of the first two peptides, and 0.09 mmole of leucine, for the third one.

For the addition of successive amino acids exactly the same protocol as described for Peptide A was followed, with the omission of the double coupling cycles, and, in the case of Peptides B and C, increasing the excess from five to seven times for the last seven and ten residues respectively. The tyrosine present in peptide C was added in the preactivated (OPfp) form, and was protected on the side chain with a tert-butyl (tBu) group.

Also regarding the cleavage from the resin and subsequent purification, the same protocol developed for Peptide A was used.

The yield of each of the synthesized peptides, measured as a percentage of the area under the peak corresponding to the desired peptide with respect to the total area of all peaks present on the HPLC trace, was about 80% for Peptide D, 78% for Peptide B, 71% for Peptide C and about 66% for Peptide A.

### EXAMPLE 2

### Antibacterial activity assay

The antibacterial activity of the peptides of this invention was evaluated by determining the minimal inhibitory concentration (MIC) of bacterial growth.

Each peptide was incubated in microtitration plates, at concentrations that decreased form 320 to 0.1 micrograms/ml, in which the wells contained a final volume of 0.1 ml of Mueller-Hinton broth with 3-5x10⁴ bacteria at the semi-logarithmic growth phase. After incubation for 16 hours at 37° C, the MIC was taken as the minimal concentration which left clear the suspension present in the wells of the microdilution plate.

In each experiment, the MIC of Bac5 and Bac7 were also measured, for the purpose of comparison.

In these assays, the following bacterial strains were used: Escherichia coli ATCC 25922, Salmonella typhimurium ATCC 14028, Klebsiella pneumoniae ATCC 13883 and Enterobacter cloacae ATCC 13047.

The results obtained are illustrated in the following Table 1.

For a more correct evaluation of the relationship between the activity of Bac7, Bac5 and the peptides of this invention, in the further Table 2 the respective MICs are expressed as micromoles.

**TABLE 1**

| Organism and strain | MIC (µg/ml) | | | | | |
|---|---|---|---|---|---|---|
| | Bac7 | A | B | D | Bac5 | C |
| Escherichia coli ATCC 25922 | 5-10 | 1.2 | 1.2-2.5 | 2.5 | 5 | 2.5-5 |
| Salmonella typhimurium ATCC 14028 | 5-10 | 0.6-1.2 | 0.6-1.2 | 1.2 | 5 | 2.5 |
| Klebsiella pneumoniae ATCC 13883 | 5 | 0.6-1.2 | 0.6-1.2 | 0.6-1.2 | 2.5-5 | 2.5 |
| Enterobacter cloacae ATCC 13047 | 5 | 0.6 | 0.6-1.2 | 1.2-2.5 | 5-10 | 2.5-5 |

**TABLE 2**

| Organism and strain | MIC (µM) | | | | | |
|---|---|---|---|---|---|---|
| | Bac7 | A | B | D | Bac5 | C |
| Escherichia coli ATCC 25922 | 0.7-1.4 | 0.3 | 0.4-0.8 | 1.0 | 1.0 | 0.8-1.6 |
| Salmonella typhimurium ATCC 14028 | 0.7-1.4 | 0.15-0.3 | 0.2-0.4 | 0.5 | 1.0 | 0.8 |
| Klebsiella pneumoniae ATCC 13883 | 0.7 | 0.15-0.3 | 0.2-0.4 | 0.2-0.5 | 0.5-1.0 | 0.8 |
| Enterobacter cloacae ATCC 13047 | 0.7 | 0.15 | 0.2-0.4 | 0.5-1.0 | 1-2 | 0.8-1.6 |

Thanks to their antibacterial activity, the compounds of formula (I) of this invention can thus be used as food preservatives, for the topical treatment of skin infections, for the disinfection of surgical instruments and in other applications well known to the persons skilled in the art.

## Claims

1. A peptide of formula
Arg-Z₁-Pro-Z₂-Arg-Pro-Z₃ (I)
wherein
Z₁ is Arg-Ile-Arg or Phe-Arg-Pro,
Z₂ is Arg-Pro-Pro-Arg-Leu-Pro or Ile-Arg-Arg-Pro-Pro-Ile,
Z₃ is Arg-Pro-Arg-Pro-Leu, Pro-Phe-Tyr-Pro-Pro-Phe-Arg-Pro, Arg-Pro-Arg-Pro-Leu-Pro-Phe-Pro-Arg-Pro, Pro-Phe-Tyr-Pro-Pro-Phe-Arg-Pro-Pro-Ile-Arg-Pro or Arg-Pro-Arg-Pro-Leu-Pro-Phe-Pro-Arg-Pro-Gly-Pro-Arg-Pro-Ile-Pro-Arg-Pro-Leu-Pro-Phe-Pro.

2. A peptide according to claim 1, wherein Z₁ is Arg-Ile-Arg, Z₂ is Arg-Pro-Pro-Arg-Leu-Pro and Z₃ is Arg-Pro-Arg-Pro-Leu-Pro-Phe-Pro-Arg-Pro.

3. A peptide according to claim 1, wherein Z₁ is Phe-Arg-Pro, Z₂ is Ile-Arg-Arg-Pro-Pro-Ile and Z₃ is Pro-Phe-Tyr-Pro-Pro-Phe-Arg-Pro-Pro-Ile-Arg-Pro.

4. A peptide according to claim 1, wherein Z₁ is Arg-Ile-Arg, Z₂ is Arg-Pro-Pro-Arg-Leu-Pro and Z₃ is Arg-Pro-Arg-Pro-Leu.

5. A peptide according to claim 1, wherein Z₁ is Arg-Ile-Arg, Z₂ is Arg-Pro-Pro-Arg-Leu-Pro and Z₃ is Arg-Pro-Arg-Pro-Leu-Pro-Phe-Pro-Arg-Pro-Gly-Pro-Arg-Pro-Ile-Pro-Arg-Pro-Leu-Pro-Phe-Pro.

6. A process for preparing a compound of formula
Arg-Z₁-Pro-Z₂-Arg- Pro-Z₃ (I)
where
Z₁ is Arg-Ile-Arg or Phe-Arg-Pro,
Z₂ is Arg-Pro-Pro-Arg-Leu-Pro or Ile-Arg-Arg-Pro-Pro-Ile,
Z₃ is Arg-Pro-Arg-Pro-Leu, Pro-Phe-Tyr-Pro-Pro-Phe-Arg-Pro, Arg-Pro-Arg-Pro-Leu-Pro-Phe-Pro-Arg-Pro, Pro-Phe-Tyr-Pro-Pro-Phe-Arg-Pro-Pro-Ile-Arg-Pro or Arg-Pro-Arg-Pro-Leu-Pro-Phe-Pro-Arg-Pro-Gly-Pro-Arg-Pro-Ile-Pro-Arg-Pro-Leu-Pro-Phe-Pro,
characterized in that the Fmoc technique on an automated continuos flow synthesizer is used.

7. A process according to claim 6, characterized in that an insoluble support, consisting of a polyamide resin is used.

8. A process according to claim 7, characterized in that the polyamide resin is polymerized on an inorganic matrix.

9. A process according to claim 8, characterized in that the inorganic matrix consists of Kieselguhr.

10. A process according to claim 8 or 9, characterized in that the C-terminal amino acid of the peptide to be synthesized, with its amino group protected by 9-flourenylmethoxycarbonyl, is attached to the insoluble support via a linker group.

11. A process according to claim 10, characterized in that successive amino acids, also protected in the same manner, are condensed via repeated cycles of deprotection in a basic environment and coupling in the presence of suitable catalysts.

12. A process according to claim 11, characterized in that the activators used in the case of non preactivated amino acids are benzotriazol-1-yl-N-oxytris(dimethylamino) phosphonium hexafluoro-phosphate coupled with 1-hydroxybenzotriazole, or 2-(1H-benzotriazol-1-yl)-1,1,3,3-tetramethyluronium tetraflouroborate.

13. A process according to claim 12, characterized in that the catalyst used in the case of amino acids preactivated as pentafluorophenyl esters is 1-hydroxybenzotriazole.

14. A process according to any one of claims 11 to 13, characterized in that the arginine residues are protected, as well as at the alpha-amino group, also on the side chain via the 2,2,5,7,8-pentamethylchroman-6-sulphonyl group.

15. A process according to any one of claims 11 to 14, characterized in that the tyrosine residues are protected, as well as at the alpha-amino group, also on the side chain via the tert-butyl group.

16. A process according to any one of claims 6 to 15, characterized in that on completion of the synthesis, the desired peptide is cleaved from the solid support with trifluoroacetic acid.

17. A process according to claim 16, characterized in that the peptide is purified via HPLC with a reverse phase column, eluting with a gradient of acetonitrile/water in the presence of trifluoroacetic acid at 0.1%.
